# EUROPEAN PATENT APPLICATION

(11) **EP 4 810 054 A1**
(43) Date of publication of application: **23.09.2026**
(21) Application number: 24891022.6
(22) Date of filing: 23.07.2024
(51) Int. Cl.: A61K 38/05, A61P 43/00, C12N 5/079, C12Q 1/06

(54) **NECROPTOSIS CONTROL AGENT**

(30) Priority: 13.11.2023 JP 2023193199
(71) Applicant: The University of Tokyo, Bunkyo-ku, Tokyo 113-8654 (JP); NH Foods Ltd., Osaka-shi, Osaka 530-0001 (JP)
(72) Inventor: HISATSUNE Tatsuhiro, Tokyo 113-8654 (JP); LEI Chenxu, Tokyo 113-8654 (JP); MATSUMOTO Takashi, Tsukuba-shi, Ibaraki 300-2646 (JP); SATO Mikako, Tsukuba-shi, Ibaraki 300-2646 (JP); SUGAWARA Yukihiro, Tsukuba-shi, Ibaraki 300-2646 (JP)
(74) Representative: Vossius & Partner Patentanwälte Rechtsanwälte mbB
(86) International application number: PCT/JP2024/026327
(87) International publication number: WO 2025/104978

(57) **Abstract**

The object is to provide relatively low-molecular-weight compounds capable of regulating necroptosis. An agent for treating a disease or condition improved by regulating necroptosis, containing a compound represented by the formula I or formula II, or a pharmaceutically acceptable salt thereof (in the formulas I and formula II, R¹, R², and R³ are each independently H or C₁₋₆ alkyl; X is H or COR⁴, where R⁴ is H, C₁₋₆ alkyl, benzyl (which may be substituted), or H₂C=CH-).

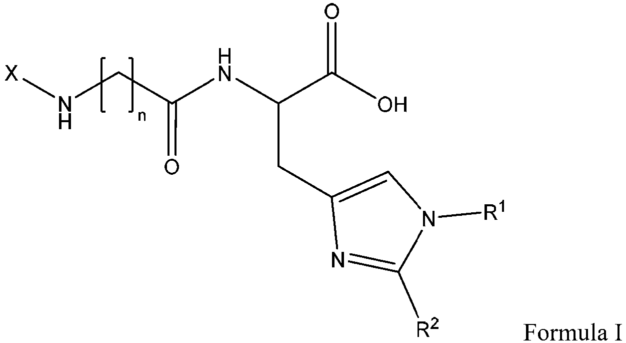

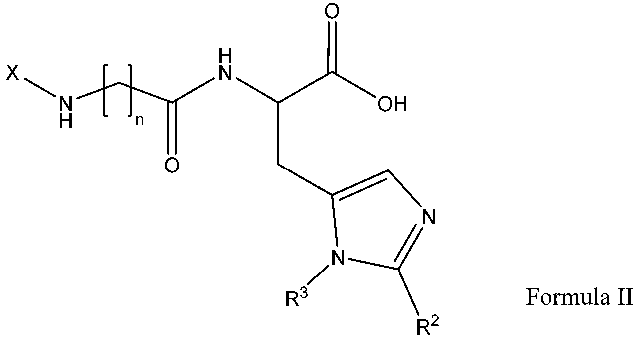

## Description

### Technical Field

The present disclosure relates to compounds capable of regulating necroptosis, and use thereof.

### Background Art

Necroptosis is one form of regulated cell death triggered by disturbances in extracellular or intracellular homeostasis, and it depends on the kinase activities of MLKL (mixed lineage kinase domain-like), RIPK3 (receptor interacting protein kinase 1), and, in at least some environments, RIPK1 (Non-patent document 1). Necroptosis can be initiated by various stimuli, such as TNF-α and Fas ligand, and in many cell types, such as monocytes, fibroblasts, lymphocytes, macrophages, epithelial cells, and neurons.

Diseases in which necroptosis is known to be involved include brain damage after stroke, myocardial cell death in myocardial infarction, acute renal failure, acute liver failure, sepsis, organ failure after organ transplantation, Alzheimer's disease, Parkinson's disease, Huntington's disease, amyotrophic lateral sclerosis; ulcerative colitis, Crohn's disease, rheumatism, autoimmune cardiomyopathy, autoimmune hepatitis, retinal degeneration, retinitis pigmentosa, chronic obstructive pulmonary disease (COPD), systemic inflammatory response syndrome (SIRS), novel coronavirus infection (COVID-19), frailty, etc.

Meanwhile, anserine and carnosine are dipeptides found at high concentrations in the skeletal muscles and brains of vertebrates, and their physiological activities have attracted attention due to their antioxidant and buffering capabilities derived from the imidazole group. For example, Patent documents 1 and 2 describe an agent containing at least one kind of imidazole dipeptide selected from the group consisting of carnosine, anserine, balenine, and homocarnosine, which agent is for inducing expression of a gene encoding at least one neurotrophic factor selected from the group consisting of BDNF, NGF, NTF-4, CSF-3, and CYR61 in glial cells, and an agent containing at least one kind of imidazole dipeptide selected from the group consisting of carnosine, anserine, balenine, and homocarnosine, which agent is for inducing secretion of exosomes having a nerve cell activation effect from intestinal epithelial cells. Further, Patent document 3 describes an oral agent containing at least one imidazole dipeptide selected from the group consisting of carnosine, anserine, balenine, and homocarnosine, which is targeting individuals aged 40 or older, and for at least one selected from the group consisting of improving age-related cognitive decline, improving age-related logical memory decline, and reducing the risk of developing dementia (excluding Alzheimer's disease).

Furthermore, regarding carnosine, it has been reported that when carnosine was continuously administered for 30 days at two doses (25 mg/kg and 50 mg/kg) to male albino rats with experimentally induced sepsis, carnosine administration significantly normalized lipid peroxidation and other antioxidant enzymes, and reduced IL-β, TNF-α, and MIF, and the carnosine treatment increased expression of IκBα and suppressed increased expression of p65, which is a protein constituting NF-κB, and p-IKKα/β (Ser 180/Ser 181) (Non-patent document 2).

### Prior Art References

### Patent documents

Patent document 1: Japanese Patent Publication (Kokai) No. 2018-30809 (Japanese Patent No. 6550357)
Patent document 2: Japanese Patent Publication (Kokai) No. 2018-83808 (Japanese Patent No. 6510004)
Patent document 3: Japanese Patent Publication (Kokai) No. 2019-70048 (Japanese Patent No. 6588666)

### Non-patent documents

Non-patent document 1: Cell Death Differ. 2018, 25(3):486-541. doi: 10.1038/s41418-017-0012-4
Non-patent document 2: Pharmacology 2017;100:292-300. DOI: 10.1159/000479879

### Summary

### Object to be achieved by the Invention

Compounds that are relatively low-molecular-weight compounds and capable of regulating necroptosis are considered to be useful as anti-necroptosis agents. Further, identifying compounds capable of regulating necroptosis and investigating optimization thereof are expected to aid in elucidating the mechanisms of necroptosis and contribute to the development of more effective anti-necroptosis agents.

Furthermore, the IκB kinase (IKK) complex, which is the master kinase of NF-κB activity, mediates NF-κB signaling by phosphorylating the IκB protein during inflammatory and immune responses. In addition, activation of the IKK complex responds to various stimuli and regulates diverse functions independently of NF-mB. If a compound capable of inhibiting the IKK/NF-κB signaling pathway is identified, it would contribute to the treatment of immune disorders and excessive inflammatory responses, as well as to the elucidation of their mechanisms.

### Means for achieving the object

The present disclosure provides the following.
[1] An agent for treating a disease or condition that is improved by regulating necroptosis, the agent comprising a compound represented by the following formula I or formula II, or a pharmaceutically acceptable salt thereof. 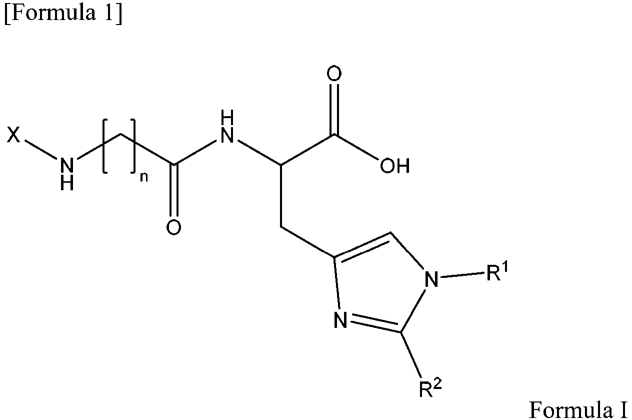 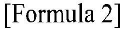 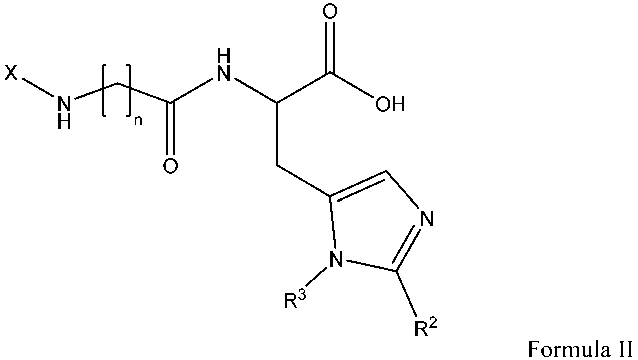 (in the formulas I and II, R¹, R²,and R³ are independently H or C₁₋₆ alkyl, n is an integer of 1 to 6; and X is H or COR⁴, where R⁴ is H, C₁₋₆ alkyl, benzyl (which may be substituted), or H₂C=CH-).
[2] The agent according to [1], wherein necroptosis is neuronal necroptosis.
[3] The agent according to [1] or [2], which regulates necroptosis-related responses in astrocytes.
[4] The agent according to any one of [1] to [3], which suppresses an increase in the expression level of any one selected from C3, Ccl2, Ccl7, Cxcl1, and iNOS.
[5] The agent according to any one of [1] to [4], which increases expression level of milk fat globule-EGF factor 8 protein (Mfge8).
[6] The agent according to any one of [1] to [5], which is for treating a subject carrying the type 4 allele of apolipoprotein E (APOE4) gene.
[7] The agent according to any one of [1] to [6], wherein the compound represented by the formula I or formula II or a pharmaceutically acceptable salt thereof is a compound represented by the formula II or a pharmaceutically acceptable salt thereof, wherein R² is H, R³ is CH₃, and n is 2.
[8] A method for screening for compounds that regulate necroptosis, using a compound represented by the formula I or formula II defined in [1] as a lead compound.
[9] An inflammatory astrocyte obtainable by treating, with TNF-α, astrocytes obtained by inducing differentiation of mouse striatal precursor-1 (MSP-1) cells.
[10] A method for screening for compounds that regulate necroptosis, using the inflammatory astrocyte according to [9].
[11] The method according to [10], which comprises treating the inflammatory astrocyte according to [9] with candidate compounds, and selecting compounds from the candidate compounds based on whether increase in expression level of a necroptosis-inducing factor is regulated.
[12] The method according to [10] or [11], which is for screening for compounds that regulate neuronal necroptosis.
[13] An agent for regulating necroptosis, the agent comprising a compound represented by the formula I or formula II defined in [1], or a pharmaceutically acceptable salt thereof.
[14] The agent according to any one of [1] to [7], which further has at least one of functions of inhibiting the NF-κB signaling pathway and inhibiting IKK activation.

### Effect of the Invention

A relatively low-molecular-weight compound capable of regulating necroptosis is provided. Use of this compound as a pharmaceutical is expected to enable treatment of diseases or conditions to be improved by regulating necroptosis.

Use of this compound as a lead compound is expected to enable optimization of compounds capable of regulating necroptosis.

### Brief Description of the Drawings

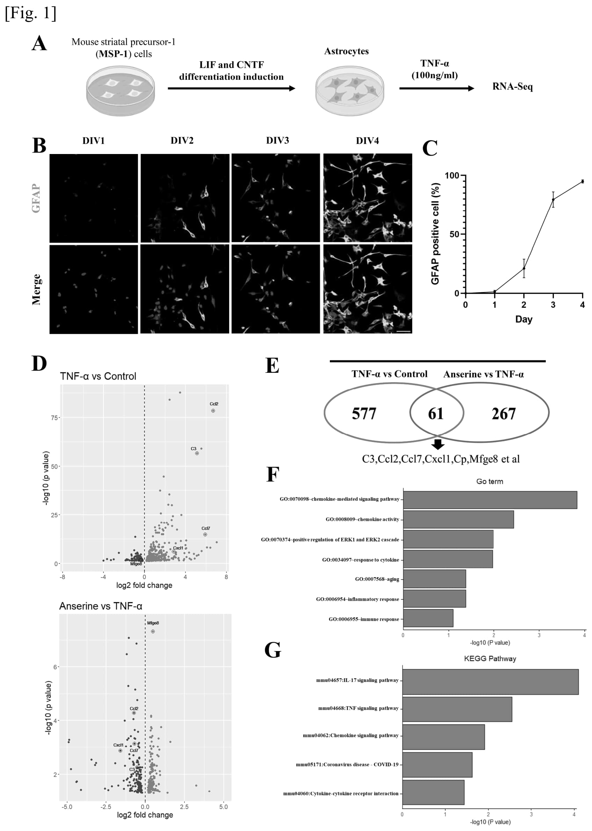

[Fig. 1] Suppression of necroptosis-related responses in astrocytes by anserine. Mouse striatal precursor cells MSP-1 can be induced to differentiate into astrocytes (Fig. 1A). After 4 days of induction, substantially all MSP-1 cells differentiated into astrocytes (Figs. 1B and 1C). When MSP-1 cells differentiated into astrocytes were treated with TNF-α and analyzed by RNA sequencing (RNA-seq), C3, Ccl2, Ccl7, and Cxcl1 were up-regulated following the TNF-α treatment, whereas they were down-regulated in the TNF-α + anserine group, where anserine was added to the culture medium prior to the TNF-α treatment. In addition, Mfge8 decreased in the TNF-α treatment group, but increased in the TNF-α + anserine treatment group (Fig. 1D and 1E). Gene ontology (GO) and Kyoto Encyclopedia of Genes and Genomes (KEGG) analyses revealed a significant decrease in the expression of genes involved in necroptosis induction in the TNF-α + anserine group (Fig. 1F and 1G).

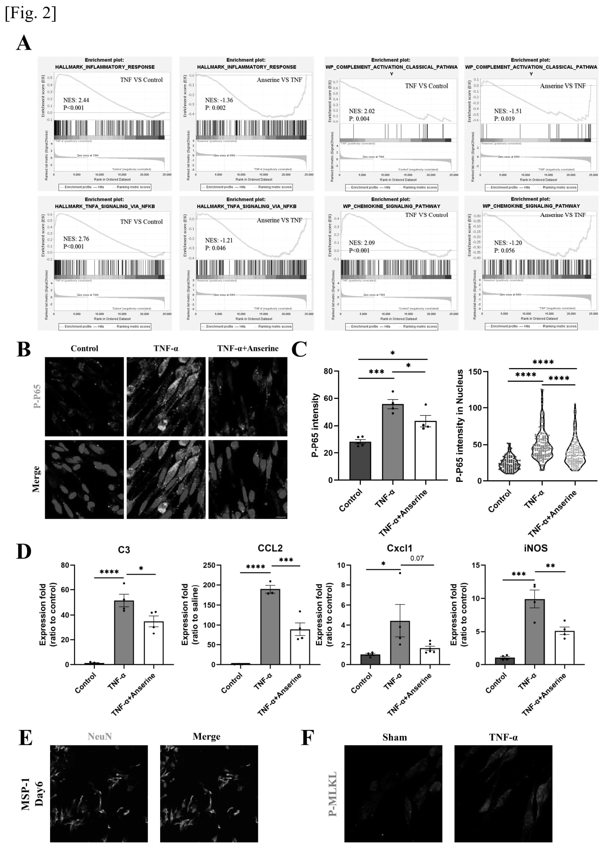

[Fig. 2] Gene set enrichment analysis (GSEA) based on whole genome expression data. In the TNF-α group, expressions of inflammation-related genes, the chemokine pathway, and the complement activation pathway were significantly increased, whereas they were decreased in the TNF-α + anserine group (Fig. 2A). Immunostaining images and quantitative data of NF-κB (P-65) in MSP-1 astrocytes following the TNF-α induction (Figs. 2B and 2C). It was confirmed that anserine treatment reduced the expression level of phospho-NF-κB1 (P-P65), and real-time PCR revealed that expressions of C3, Ccl2, Cxcl1, and iNOS were significantly increased in the TNF-α treatment group, whereas they were significantly decreased in the anserine treatment group (Fig. 2D). When MSP-1 cells were induced to differentiate into neurons (Fig. 2E), and treated with the culture supernatant of the TNF-α-treated MSP-1 astrocytes, the expression level of phosphorylated MLKL (P-MLKL) increased in the treated group compared with the untreated group (Fig. 2F). For Figs. 2C and 2D, *p < 0.05; **p < 0.01; ***p < 0.001; and ****p < 0.0001.

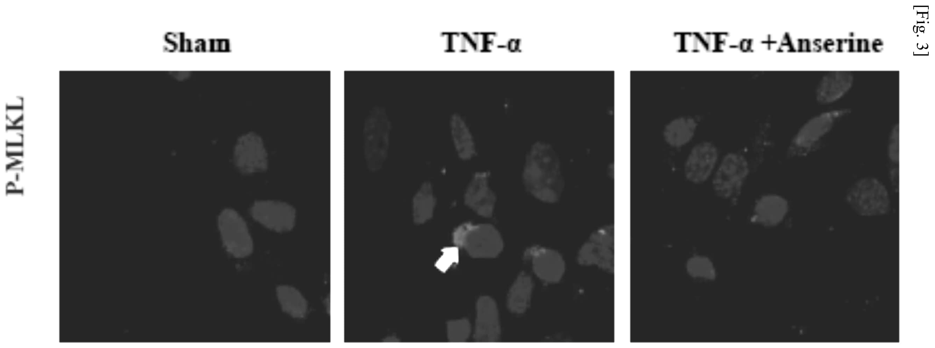

[Fig. 3] Necroptosis-protective effect of anserine on neurons. MSP-1 neurons treated with the culture supernatant of the TNF-α-treated astrocytes underwent necroptosis accompanied by the expression of P-MLKL in necrosomes (indicated by the arrow in the figure) (TNF-α group). In contrast, when neurons were treated with culture supernatant obtained by adding anserine during the TNF-α treatment (TNF-α + anserine group), necroptosis was suppressed to the same extent as when treated with the culture supernatant obtained without the treatment (sham group).

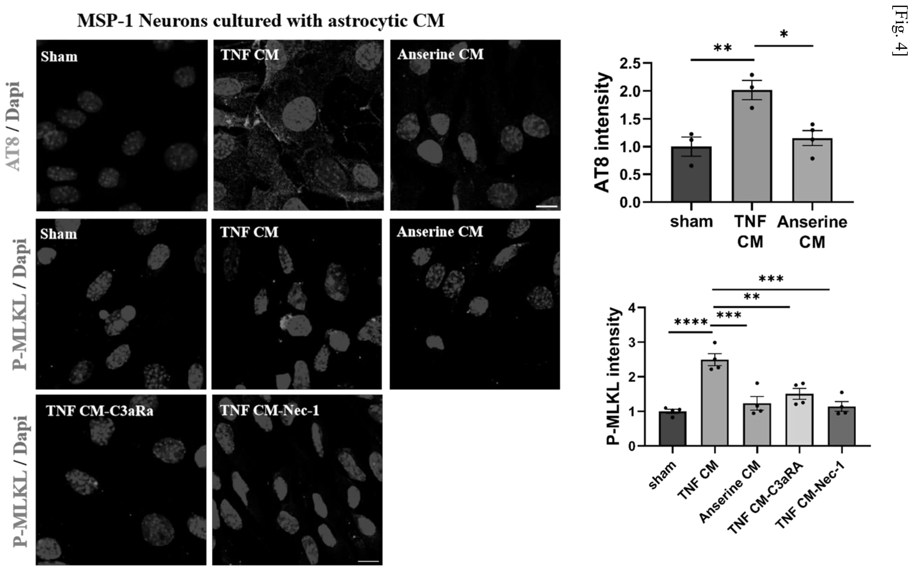

[Fig. 4] Comparison of anserine with necroptosis inhibitors and C3a receptor antagonists. Reproduced images of AT8 staining of MSP-1 neurons of sham and those treated with TNF CM, and Anserine CM (scale bar, 10 µm). Quantification of AT8 intensity in each group (n=4). Representative images of P-MLKL staining of MSP-1 neurons of sham and those treated with TNF CM, anserine CM, TNF CM-C3aRA (10 µM), and TNF CM-Nec 1 (30 µM) (scale bar, 10 µm). Quantification of P-MLKL staining in each group (n=4). The data represent mean ± SEM and were analyzed by one-way ANOVA based on Tukey's multiple comparison test. *P < 0.05; **P < 0.001; ***P < 0.005; and ****P < 0.0001. CM, culture medium; Nec-1, necroptosis inhibitor; and C3aRA, C3aR antagonist.

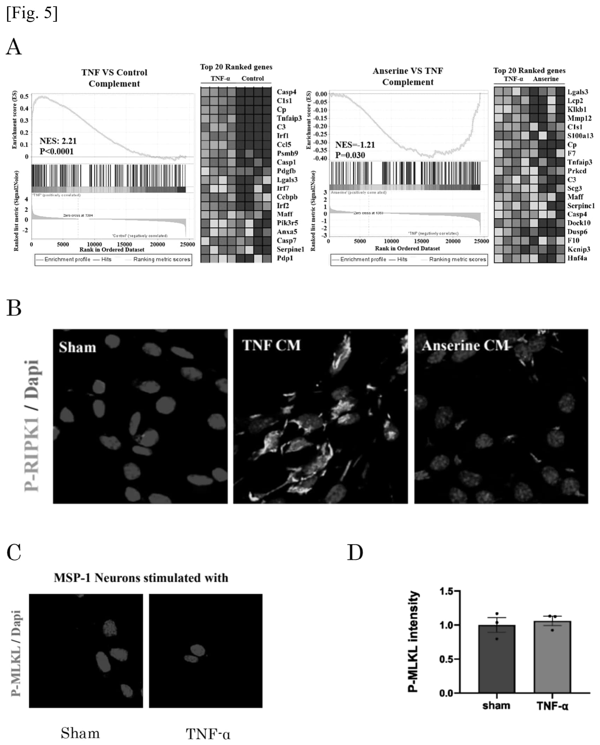

[Fig. 5] MSP-1 astrocytes pretreated with anserine exhibited reduced NF-κB activity and complement activation, and consequently, decreased expression of inflammatory factors such as C3, which had been observed to increase in the TNF-α treatment group (Fig. 5A). Staining using necroptosis markers revealed that neurons treated with TNF-α CM exhibited high intensities of P-RIPK1 and P-MLKL (Figs. 4 and 5B), indicating that necroptosis was activated. In contrast, direct treatment of MSP-1 neurons with TNF-α did not induce P-MLKL expression (Figs. 5C and 5D).

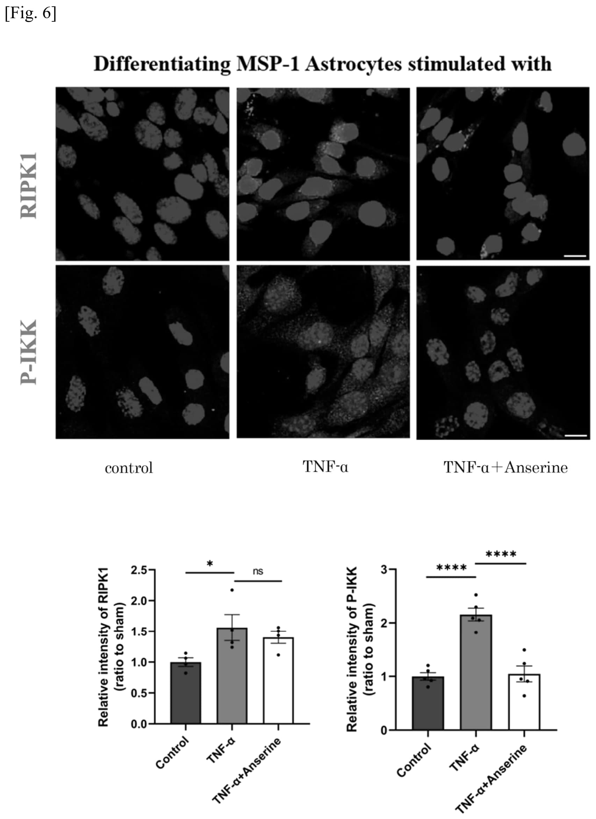

[Fig. 6] Inhibition of IKK activation by anserine. In MSP-1 astrocytes treated with TNF-α, increases in RIPK1 and phosphorylated IKK were observed; however, the addition of anserine reduced the expression of phosphorylated IKK. It is believed that anserine suppresses the neurotoxicity of TNF-α-induced astrocytes in vitro. Consequently, C3 release from astrocytes may be suppressed, which may suppress necroptosis in neurons.

### Detailed Description

### [Regulation of necroptosis, inhibition of NF-κB signaling pathway, and inhibition of IKK activation]

### (Active ingredient)

The composition of this embodiment contains, as an active ingredient, a compound represented by the following formula I or formula II, or a pharmaceutically acceptable salt thereof.

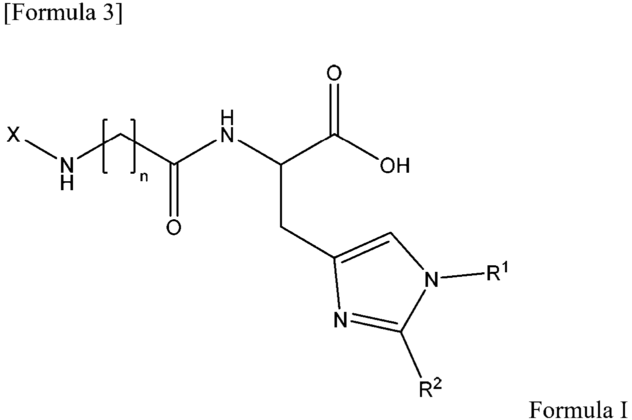

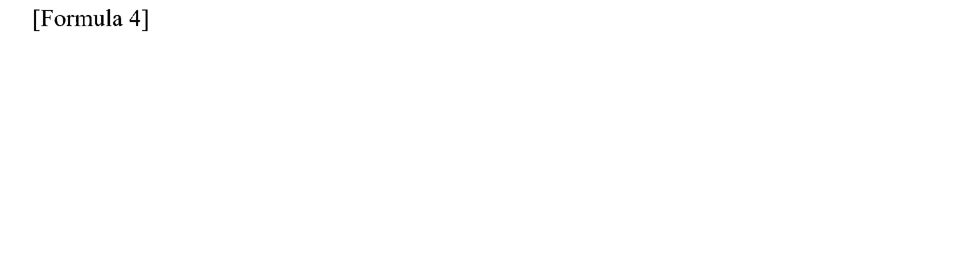

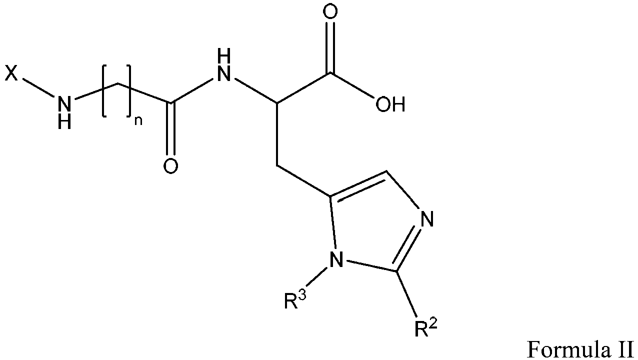

In the formulas I and II, R¹, R², and R³ are each independently H or C₁₋₆ alkyl, n is an integer of 1 to 6, and X is H or COR⁴, where R⁴ is H, C₁₋₆ alkyl, benzyl (which may be substituted), or H₂C=CH-.

In the formula I, it is preferred that one of R¹and R² is C₁₋₆ alkyl and the other is H. In the formula II, it is preferred that one of R² and R³ is C₁₋₆ alkyl and the other is H. A preferred example of C₁₋₆ alkyl is methyl. In either case, n is preferably an integer of 2 to 5, more preferably 2 or 3.

When X is -COR⁴, specific examples of X include formyl, acetyl, propionyl, benzoyl, and acryloyl.

In one embodiment, the active ingredient is a compound represented by the formula II or a pharmaceutically acceptable salt thereof, wherein R² is H, R³ is CH₃, and n is 2. Such a compound is anserine or a pharmaceutically acceptable salt thereof. In the present disclosure and embodiments thereof, anserine may be either the L-isomer or D-isomer thereof, unless especially specified,. The same applies to the other compounds.

The structure of L-anserine (IUPAC name: (2S)-2-[(3-amino-1-oxopropyl)amino]-3-(3-methyl-4-imidazolyl)propanoic acid) is shown below.

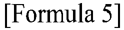

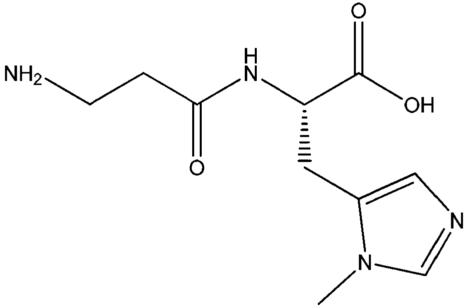

In another embodiment, the active ingredient is any selected from the group consisting of carnosine, balenine, and homocarnosine, or a pharmaceutically acceptable salt thereof.

In describing the present disclosure and embodiments thereof, examples may be given using anserine or salts thereof among the compounds represented by the formula I or formula II and their pharmaceutically acceptable salts; however, those skilled in the art will be able to apply and understand such descriptions to cases involving other compounds and salts thereof as well.

Examples of pharmaceutically acceptable salts include hydrochlorides, hydrobromides, hydroiodides, nitrates, phosphates, sulfates, formates, acetates, citrates, oxalates, fumarates, maleates, succinates, malates, tartrates, trichloroacetates, trifluoroacetates, methanesulfonates, benzenesulfonates, p-toluenesulfonates, mesitylenesulfonates, naphthalenesulfonates, alkali metal salts (e.g., sodium salts and potassium salts), alkaline earth metal salts (e.g., magnesium salts and calcium salts), ammonium salts, and mono-,di- or tri-lower alkyl or hydroxyalkyl ammonium salts (e.g., ethanol ammonium salts, diethanol ammonium salts, triethanol ammonium salts, and tromethamine salts). The salts may be anhydrids or solvates, and solvates include hydrates, methanol solvates, ethanol solvates, propanol solvates, and 2-propanol solvates.

In a preferred embodiment, the agent comprises any of the hydrochlorides, nitrates, and acetates of the compounds represented by the formula I or formula II.

### (Uses and functions)

### <Regulation of necroptosis>

The agent of the present embodiment can be used to regulate necroptosis. Regulation includes upward regulation (increase, promotion, suppression of decrease, etc.) and downward regulation (decrease, suppression, inhibition of increase, etc.).

Necroptosis is one form of regulated cell death triggered by disturbances in extracellular or intracellular homeostasis. Necroptosis is observed in many cell types, such as monocytes, fibroblasts, lymphocytes, macrophages, epithelial cells, and neurons.

Furthermore, the agent of the present embodiment may be used to treat diseases or conditions that are improved by regulating necroptosis. Examples of diseases involving necroptosis include brain injury after stroke, myocardial cell death in myocardial infarction, acute renal failure, acute liver failure, sepsis, organ failure after organ transplantation, Alzheimer's disease, Parkinson's disease, Huntington's disease, amyotrophic lateral sclerosis, ulcerative colitis, Crohn's disease, rheumatism, autoimmune cardiomyopathy, autoimmune hepatitis, retinal degeneration, retinitis pigmentosa, chronic obstructive pulmonary disease (COPD), systemic inflammatory response syndrome (SIRS), novel coronavirus infection (COVID-19), frailty, etc. The following references demonstrate the association between these diseases and necroptosis.
Non-patent document 3: Experimental Gerontology. 2017, 87:160-167. http://dx.doi.org/10.1016/j.exger.2016.05.004
Non-patent document 4: Front. Pharmacol. 2021, 12. https://doi.org/10.3389/fphar.2021.701564
Non-patent document 5: J Cent South Univ (Med Sci). 2022, 47(9), 1289. DOI: 10.11817/j.issn.1672-7347.2022.210501
Non-patent document 6: Biomolecules 2023, 13, 482. https://doi.org/10.3390/biom13030482

The agent of the present embodiment can suppress rapid progression of inflammation by inhibiting necroptosis. Therefore, it can be particularly effectively used for urgent treatment in diseases or conditions where inflammatory reactions progress rapidly and lead to death, such as brain damage after stroke, myocardial cell death after myocardial infarction, acute renal failure, sepsis, organ failure after organ transplantation, and systemic inflammatory response syndrome. In another embodiment, the agent is also expected to be effective for chronic diseases or conditions requiring long-term treatment or management.

In one embodiment, the agent is suitably used for the treatment of brain damage after stroke, myocardial cell death after myocardial infarction, acute renal failure, acute liver failure, sepsis, organ failure after organ transplantation, amyotrophic lateral sclerosis, ulcerative colitis, Crohn's disease, rheumatism, autoimmune cardiomyopathy, autoimmune hepatitis, chronic obstructive pulmonary disease (COPD), systemic inflammatory response syndrome (SIRS), and frailty.

In the context of the present disclosure or embodiments thereof, the term improvement or treatment of a disease or condition includes reduction of the risk of onset, delay of onset, prevention, treatment, and halting or delaying progression.

In one embodiment, the agent may be used to regulate necroptosis in neurons (nerve cells).

In another embodiment, the agent is one for regulating necroptosis-related responses in astrocytes. Such necroptosis-related responses in astrocytes may include increased expression levels of necroptosis-promoting or related factors, such as C3, Ccl2, Ccl7, Cxcll, and iNOS (inducible nitric oxide synthase).

In another embodiment, the agent is one for increasing the expression level of Mfge8 (milk fat globule-EGF factor 8 protein). Mfge8 is known as a factor that is mainly secreted by macrophages and specifically recognizes molecules expressed on the cell membrane upon induction of cell death. It is known that the C1C2 domain, located in the C-terminal region of Mfge8, binds to phosphatidylserine, which is exposed on the cell membranes upon necroptosis induction. Since phosphatidylserine is presumed to play an essential role in the induction of cell death accompanied by cell membrane disruption via necroptosis, it can be considered that Mgfe8 suppresses cell death caused by necroptosis by binding to phosphatidylserine to inhibit the cell death-inducing function thereof.

Whether a certain compound is capable of regulating necroptosis may be evaluated as follows.

MSP-1 cells are of a neural stem cell line established from the ventral telencephalon tissue of p53-knockout (p53-KO) mice. They can be differentiated into astrocytes by culturing them in a specific medium containing leukemia inhibitory factor (LIF) and ciliary neurotrophic factor. Further, MSP-1 cells can be differentiated into neurons by culturing them in a specific medium that does not contain these factors. According to the investigations of the inventors of the present disclosure, astrocytes differentiated from MSP-1 cells can be treated with TNF-α to obtain inflammatory glial cells. Furthermore, since the culture supernatant of inflammatory glial cells contains necroptosis-promoting factors, administering this culture supernatant to neurons differentiated from MSP-1 cells increases the expression levels of phosphorylated MLKL (P-MLKL), which is strongly associated with the induction of necroptosis.

Therefore, by co-incubating a compound desired to be evaluated with MSP-1 astrocytes during the treatment with TNF-α and determining whether it suppresses the increase in the expression level of necroptosis-promoting factors, it is possible to assess whether that compound can regulate necroptosis. Alternatively, by co-incubating a compound desired to be evaluated with MSP-1 astrocytes during the treatment with TNF-α, administering the resulting culture supernatant to MSP-1 neurons, and determining whether or not the increase in P-MLKL expression is suppressed, it can be determined whether the compound is capable of regulating necroptosis.

Therefore, the present disclosure provides, as one aspect, the following:
Inflammatory astrocytes obtainable by treating, with TNF-α, astrocytes obtained by inducing differentiation of mouse striatal precursor-1 (MSP-1) cells.

A method for screening for compounds that regulate necroptosis using the aforementioned inflammatory astrocytes. Specifically, this method comprises the step of exposing the aforementioned inflammatory astrocytes to candidate compounds and selecting compounds from the candidate compounds based on whether the increase in the expression levels of necroptosis-inducing factors is regulated. This method is particularly suitable for screening for compounds that regulate neuronal necroptosis.

### <Inhibition of NF-κB signaling pathway and inhibition of IKK activation>

NF-κB (nuclear factor-kappa B) is a protein complex that functions as a transcription factor. In the unstimulated state, NF-κB is located in the cytoplasm and is activated by migrating into the nucleus upon stimulation. When unstimulated, NF-κB binds to inhibitory proteins, such as IκBα, a member of the IκB (inhibitor of κB) family, and remains in the cytoplasm. When cells are stimulated by a specific ligand, the IKK (IκB kinase) complex is activated, causing the inhibitory proteins (such as IκBα) to be phosphorylated and degraded. The degradation of IκBα releases NF-κB from the inhibitory proteins, exposing the nuclear translocation signal that has been masked by the inhibitory proteins. NF-κB then translocates to the nucleus and activates the transcription of various genes. The IKK complex (also referred to simply as IKK for the present disclosure) consists of three types of proteins: IKK1 (IKKα), IKK2 (IKKβ), and NEMO (IKKγ).

According to the investigation of the inventors, the active ingredient of the agent of the present embodiment has a function of inhibiting the NF-κB signaling pathway. That is, the present disclosure provides an NF-κB signaling pathway inhibitor containing the active ingredient. Inhibition of the NF-κB signaling pathway refers to the inhibition of the pathway in which NF-κB is released from inhibitory proteins, the nuclear translocation signal that has been masked by the inhibitory proteins is exposed, and NF-κB translocates to the nucleus and activates the transcription of various genes. The active ingredient is thought to inhibit the NF-κB signaling pathway by inhibiting the activation of the IKK complex. NF-κB is inherently important for normal immune responses, but its sustained overexpression can lead to immune dysfunction and excessive inflammation. NF-κB is also involved in autoimmune diseases, cancers, chronic inflammatory diseases, and aging.

In one embodiment, the agent is intended to inhibit the NF-κB signaling pathway in the brain, and more specifically, it is intended to inhibit the NF-κB signaling pathway in astrocytes.

According to the investigation of the inventors, the active ingredient of the agent of the present embodiment has a function of inhibiting the activation of the IKK complex. That is, the present disclosure provides an IKK complex activation inhibitor containing the active ingredient. The IKK complex, which is the master kinase of NF-κB activation, comprises two kinase subunits, IKKα and IKKβ. In addition to mediating NF-κB signaling by phosphorylating the IκB protein during inflammatory and immune responses, the activation of the IKK complex is known to regulate various functions independently from NF-κB.

Both IKKα and IKKβ are known to phosphorylate a growing list of substrates involved in various biological functions, including tumor suppression, immune function, cell proliferation, and chromatin remodeling. Although IKKα and IKKβ are structurally and biochemically similar, they play distinct physiological and pathological roles due to differences in their intracellular localization and phosphorylation targets. IKKα is known to shuttle between the cytoplasm and the nucleus, whereas IKKβ is primarily located in the cytoplasm.

The IKK complex has been implicated in carcinogenesis, immunity, transcriptional regulation, cell cycle, crosstalk between signaling pathways, and diabetes (Immunol Rev. 2012 March; 246(1): 239-253. doi:10.1111/j.1600-065X.2012.01107.x.). Further, compounds being developed as IKKα- and IKKβ-specific inhibitors have been suggested to have efficacy against various diseases in animal models. Such diseases include myocardial infarction, pulmonary diseases, skin diseases, cancers, arthritis, rheumatoid arthritis, etc.

In one embodiment, the agent is intended to inhibit the activation of the IKK complex in the brain, more specifically, to inhibit the activation of the IKK complex in astrocytes.

### <Subject>

In one embodiment, the agent of the present embodiment is suitable for administration to subjects requiring regulation of necroptosis, or subjects with diseases or conditions that are improved by regulating necroptosis. The subjects are mammals, preferably humans (individuals).

The age and gender of the subject are not particularly limited, and the subject may be, for example, 15 years of age or older, 20 years of age or older, 40 years of age or older, an elderly person (e.g., 65 years of age or older), a child (7 years of age or older but under 15 years of age), a toddler (1 to 6 years of age), an infant (under 1 year of age), or a newborn (within 28 days of birth), and may be either male or female.

In a particularly preferred embodiment, the agent is used to treat subjects who have the apolipoprotein E gene type 4 allele APOE4. Apolipoprotein E is a protein that binds to lipoproteins to be involved in lipid metabolism, and its genetic polymorphism ε4 (APOE4 or APOEε4) is known to be a strong risk factor for the onset of Alzheimer's disease.

Arnaud et al. demonstrated that APOE regulates inflammation in human astrocytes by regulating the expression of transglelin 3 (TAGLN3), ultimately regulating NF-κB activation, and revealed that APOE4 specifically down-regulates transglelin 3 (TAGLN3) by participating in histone deacetylase activity, thereby inducing low-grade chronic inflammation and enhanced inflammatory responses (Cell Rep. 2022 Aug 16;40(7):111200. doi: 10.1016/j.celrep.2022.111200.). APOE has a function of regulating TAGLN3, while TAGLN3 acts to suppress NF-κB, whereas APOE4 reduces TAGLN3, thereby inducing NF-κB-mediated inflammation. That is, subjects carrying APOE4 are prone to the induction of NF-xB-mediated inflammation. Therefore, in one embodiment, the agent having the NF-κB signaling pathway inhibitory function is particularly suitable for treating subjects carrying APOE4.

There are mainly three types of the APOE gene, ε2, ε3, and ε4, and genotypes include ε2/ε3, ε3/ε3, ε2/ε4, ε3/ε4, ε4/ε4, etc. with the risk of developing Alzheimer's disease increasing in that order. Therefore, in one embodiment, the agent of the present embodiment is suitable for treating subjects with such genotypes as ε2/ε4, ε3/ε4, and ε4/ε4, and more specifically, it is particularly suitable for treating subjects with the genotype ε4/ε4. Blood or other samples may be collected from the subject, an APOE genetic test may be performed, and the agent of the present embodiment may be administered to subjects with the aforementioned genotypes.

### (Production method)

The active ingredient of the agent of the present embodiment may be synthetic or derived from natural sources. When using a substance derived from natural sources, it may be isolated or purified, or it may be included as an extract, concentrate, or crude preparation derived from the natural sources. Anserine is known to be abundant in bonito, tuna, salmon, and chicken.

Compounds represented by the formula I or formula II can be produced by known methods. The production methods are described in Japanese Patent Publications (Kokai or Kohyo), JP 2003-520221, JP 2006-232686, JP 2006-504701, JP 2008-517911, JP 2009-512459, JP 2010-31004, JP 2011-37891, JP 2011-37892, JP 2013-165728, JP 2014-12735, etc.

The agent of the present embodiment can be produced by using various known techniques. Those skilled in the art can appropriately design the production process by considering the solubility, stability, volatility etc. of the active ingredient. According to the investigation of the inventors, anserine has been confirmed to be sufficiently stable at room temperature and under conditions of 180°C or lower. It has also been confirmed that it can be stored stably in solution for at least two years and nine months.

### (Dosage form, method of use, dose etc.)

In one embodiment, the agent can be used as a pharmaceutical, and it is preferably a pharmaceutical. The agent may be referred to as a composition. The dosage form of the agent of the present embodiment is not particularly limited, but in one embodiment, it is in a dosage form suitable for oral administration. Examples of dosage forms for oral administration include tablets (including tablets with conventional coatings, sugar-coated tablets, sublingual tablets, and buccal tablets), capsules, granules, powders, fine granules, syrups (including dry syrups), enteric-coated tablets, sustained-release capsules, cachet (including wafer capsules), chewable tablets, drops, pills (excluding tablets), oral liquids (including all liquid formulations to be administered orally), confectionery tablets (lozenges, candies etc.), sustained-release tablets, and sustained-release granules. Examples of other dosage forms include injections (including those dissolved at the time of use, including parenteral nutrition solutions), dusting powders, lotions, ointments and creams, shampoos, sprays, topical solutions (including liniments), tape dressings (including poultices), aerosols (metered-dose inhalers), ear drops, eye drops, eye ointments, nasal agents (including nasal sprays), inhalants (including inhalation anesthetics and inhalation sprays), inhaled gases (such as nitrous oxide), spin caps, mouthwash, rectal suppositories, insertable preparations (vaginal suppositories, vaginal tablets, etc.), enemas, jellies, and infusions (peritoneal dialysis solutions, etc.).

The genet of the present embodiment may contain components other than the active ingredient, so long as the agent can provide the desired effect. Such other components may be various pharmaceutically acceptable additives. Examples include excipients, antioxidants, fragrances, flavorings, sweeteners, colorants, thickening and stabilizing agents, color developers, bleaching agents, antifungal agents, gum bases, bittering agents, enzymes, glossing agents, acidulants, emulsifiers, fortifying agents, manufacturing aids, binding agents, tonicizing agents (isotonic agents), buffers, solubilizing agents, preservatives, stabilizers, and coagulants.

The dose of the agent of the present embodiment can be appropriately determined by those skilled in the art depending on the age, weight, gender, disease or condition to be treated of the subject, etc. The dose of the active ingredient can be, for example, 200 mg/day or more, and it is preferably 400 mg/day or more, more preferably 500 mg/day or more, further preferably 750 mg/day or more. Further, the dose may be 1,000 mg/day or more, 2,000 mg/day or more, 5,000 mg/day or more, or 7,500 mg/day or more. In any case, the dose may be 10,000 mg/day or less. Furthermore, regardless of how the minimum dose is defined, the dose may be 50,000 mg/day or less, preferably 30,000 mg/day or less, more preferably 20,000 mg/day or less, further preferably 10,000 mg/day or less. Such a daily dose may be administered in a single dose or multiple times as divided portions. The time of the administration is not particularly limited, and in the case of oral administration, it may be administered before, after, or between meals, and if symptoms change, it may be administered when symptoms are present.

The agent of the present embodiment may be repeatedly administered and may also be administered over a long period of time.

### [Use as lead compound]

One aspect of the present disclosure relates to a method for screening for compounds that regulate necroptosis using compounds represented by the formula I or II defined above as lead compounds.

The term lead compound generally refers to a compound whose pharmacological activity profile is well-defined and for which an increase in activity and a reduction in toxicity can be expected through chemical modification. The compounds represented by the formula I or II have a necroptosis-regulating activity as described above, and further chemical modification of these compounds is expected to result in increased activity and reduced toxicity.

The term "chemically modifying" refers to, for example, optimizing the lead compounds by chemically modifying them. Chemical modification may include, for example, substitution or removal of certain amino acids, or addition or insertion of at least one amino acid. Further, chemical modification may involve addition, substitution, or removal of functional groups to or from individual amino acids, as well as replacement of individual amino acids with D-isomers thereof or with synthetic amino acids.

According to the present embodiment, by performing optimization using compounds represented by the formula I or II defined above as lead compounds, active ingredients with superior properties in terms of physical properties, pharmacokinetics, and toxicity can be screened for.

### Examples

### [Example 1]

### [Method]

### (Culture of mouse striatal precursor cells (MSP-1 cells))

The MSP-1 cell line is a neural stem cell line established from ventral telencephalon tissue of p531-KO mice. MSP-1 cells were cultured in dishes coated with poly-L-lysine/fibronectin (Sigma) using DMEM/F12 medium (Gibco) containing 10% FBS (Gibco) and basic fibroblast growth factor (basic-FGF, 10 ng/ml, Fujifilm Wako Pure Chemical Industries). This medium is referred to as MSP-1 proliferation medium. The culture medium was replaced with fresh medium every 2 days.

To obtain differentiated astrocytes, the above MSP-1 cells were harvested and cultured overnight in the MSP-1 proliferation medium on new dishes. The next day, for differentiation into astrocytes, the cells were cultured in serum-free DMEM/F12 medium containing N-2 supplement (R&D Systems), leukemia inhibitory factor (LIF, 10 ng/ml, Fujifilm Wako Pure Chemical Industries), and ciliary neurotrophic factor (CNTF, 10 ng/ml, Fujifilm Wako Pure Chemical Industries). For differentiation into neurons, the cells were cultured in serum-free DMEM/F12 medium containing only N-2 supplement. These media are referred to as MSP-1 differentiation media. Each medium was replaced with fresh medium every 2 days. For differentiation into astrocytes, in particular, on day 4, the cells were exposed to tumor necrosis factor-α (TNF-α, 100 ng/ml, Fujifilm Wako Pure Chemical Industries) for 24 hours. In the anserine treatment group, the cells were pretreated with L-anserine nitrate (Fujifilm Wako Pure Chemical Industries) for 2 hours prior to the exposure to TNF-α.

### (Immunocytochemical staining)

The harvested MSP-1 cells were cultured in the MSP-1 differentiation medium on poly-L-lysine/fibronectin-coated chamber glass (4-well, Watson) and differentiated into astrocytes. After the TNF-α treatment, the cells were fixed with 4% PFA for 10 minutes and washed with TBS for 10 minutes. Subsequently, the cells were blocked with 5% BSA diluted in 0.05% TBS-X at room temperature for 60 minutes, then incubated overnight at 4°C in a blocking solution containing primary antibodies. The primary antibodies used in this study were anti-GFAP (mouse, 1:1000, Sigma), anti-NeuN (mouse, 1:1000, Millipore), anti-NF-κB p65 (rabbit, 1:500, CST), and anti-P-MLKL (rabbit, 1:2000, Abcam). The cells were then washed three times with TBS for 5 minutes each, and incubated with secondary antibodies at room temperature for 2 hours. The secondary antibodies used in this study were anti-mouse IgG donkey Alexa 488 (1:1000, Molecular Probes) and anti-rabbit IgG donkey Alexa 568 (1:1000, Molecular Probes). After washing with TBS for 5 minutes, the sections were incubated with DAPI (1:10,000, Sigma) in TBS for 5 minutes, and then washed with TBS 2 times for 5 minutes each. The cells were visualized using a confocal microscope (FV3000-L4EN-TN21, Olympus).

### (RNA sequencing)

The MSP-1 cells differentiated into astrocytes were harvested by trypsin treatment, and total RNA was isolated using the RNeasy Mini Kit (Qiagen). Samples (10 ng input) were pretreated using the SMART-seq Stranded Kit (Takara, Japan). Sequencing was performed using Illumina NovaSeq 6000 (Illumina, USA).

Gene expression levels were assessed by using transcripts per million (TPM) data, and differentially expressed genes (DEGs) were identified using the DESeq2 package in the R environment. Genes with p-values less than 0.05 were considered DEGs. Down-regulated genes (Anserine vs. TNF-α) were clustered, and the gene set from Cluster 1 was used for gene ontology (GO) analysis and Kyoto Encyclopedia of Genes and Genomes (KEGG) pathway analysis. Gene set enrichment analysis (GSEA) was performed based on the TPM data.

### [Results]

### Anserine suppressed necroptosis-related responses in astrocytes

To determine whether anserine has an activity for suppressing necroptosis-related activation responses in astrocytes, cultured astrocytes were used to investigate whether anserine has an activity for inhibiting the production of necroptosis-inducing cytokines mediated by astrocytes. For the experiment, MSP-1 cells, i.e., mouse striatal precursor cells, were used. The MSP-1 cells can be induced to differentiate into astrocytes (Fig. 1A). After 4 days of induction, it was found that substantially all cells had become astrocytes (Figs. 1B and 1C).

On day 5, TNF-α (100 ng/ml) was added to the MSP-1 cells differentiated into astrocytes, and the cells were treated for 24 hours. In the TNF-α + anserine group, 10 µM anserine was added to the culture medium 2 hours prior to the TNF-α treatment. These cells were harvested, and RNA sequencing (RNA-seq) analysis was performed to examine the expressed genes. As a result, 638 differentially expressed genes (DEGs) were identified in the TNF-α treatment group in comparison with the control group (458 up-regulated, 180 down-regulated), and 328 DEGs were identified in the TNF-α + anserine treatment group in comparison with the TNF-α treatment group (159 up-regulated, 169 down-regulated). Necroptosis-promoting factors such as C3, Ccl2, Ccl7, and Cxcl1 increased after the TNF-α treatment and decreased in the TNF-α + anserine group. Further, Mfge8 (milk fat globule-EGF factor 8), which is involved in the suppression of necroptosis, was found to have decreased in the TNF-α treatment group but increased in the TNF-α + anserine treatment group (Figs. 1D and 1E).

Furthermore, gene ontology (GO) and Kyoto Encyclopedia of Genes and Genomes (KEGG) analyses were performed. In the TNF-α + anserine group, significant decrease in expression was observed in the gene groups involved in necroptosis induction (chemokine signaling pathway, response to cytokine, immune response, and TNF signaling-related genes) (Figs. 1F and 1G).

Gene set enrichment analysis (GSEA) was also performed based on the expression data of all genes. In the TNF-α group, the expressions of inflammation-related genes, chemokine pathway, and complement activation pathway significantly increased, whereas in the TNF-α + anserine group, they were decreased (Fig. 2A). This may be associated with decrease in NF-κB signaling (Fig. 2A). It was confirmed that the anserine treatment reduced the expression of phospho-NF-κB (Figs. 2B and 2C). Real-time PCR revealed that in the TNF-α treatment group, the expressions of C3, Ccl2, Cxcl1, and iNOS significantly increased, whereas in the anserine treatment group, the expression of these genes significantly decreased (Fig. 2D).

### Anserine consequently suppressed necroptosis of neurons

The MSP-1 cells were induced to differentiate into neurons (Fig. 2E). In the group treated with the culture supernatant from TNF-α-treated MSP-1 astrocytes, the expression level of phosphorylated MLKL (P-MLKL), which is most closely associated with the induction of necroptosis, was found to be elevated compared with the untreated group (Fig. 2F).

Furthermore, as shown in Fig. 3, it was found that MSP-1 neurons treated with the culture supernatant from TNF-α-treated astrocytes (TNF-α group) exhibited necroptosis, accompanied by the expression of P-MLKL in necrosomes (indicated with an arrow in the figure); however, when the cells were treated with culture supernatant containing 10 mM anserine in addition to TNF-α (TNF-α + anserine group), necroptosis was suppressed to a degree comparable to that observed with untreated culture supernatant (sham group).

These results strongly suggest that anserine prevents necroptosis in neurons mediated by astrocytes.

### [Example 2]

### [Method]

### (Culture of mouse striatal precursor-1 (MSP-1) cells)

The MSP-1 cell line was obtained from a neural stem cell line established from ventral telencephalon tissue of p53 KO mice. The MSP-1 cells were cultured in poly-L-lysine/fibronectin-coated dishes (Sigma) using DMEM/F12 medium (Gibco) containing 10% FBS (Gibco) and basic fibroblast growth factor (basic-FGF, 10 ng/ml, Wako). This medium was referred to as MSP-1 proliferation medium. The culture medium was replaced with fresh medium every 2 days.

To obtain differentiated astrocytes, the MSP-1 cells were harvested and cultured overnight in the MSP-1 proliferation medium on new dishes. The next day, the medium was replaced with serum-free DMEM/F12 medium containing N-2 supplement (R&D Systems), leukemia inhibitory factor (LIF, 10 ng/ml, Wako), and ciliary neurotrophic factor (CNTF, 10 ng/ml, Wako). This medium was referred to as astrocyte differentiation medium. The culture medium was replaced with fresh medium every 2 or 3 days. On day 5, the cells were exposed to tumor necrosis factor-α (TNF-α, 100 ng/ml, Wako) for 24 hours. For anserine treatment group, the cells were pretreated with L-anserine (Toronto Research Chemicals) for 2 hours prior to the exposure to TNF-α. To obtain differentiated neurons, the culture medium for MSP-1 cells was replaced with the serum-free DMEM/F12 medium containing N-2 supplement. This medium was referred to as neuron differentiation medium. The culture medium was replaced with fresh medium every 2 days. On day 5, the cells were treated for 24 hours with the culture supernatant of the astrocytes induced from MSP-1 cells. Nec-1 and the C3aR antagonist (SB290157) were purchased from Selleck.

### (Immunocytochemical staining)

The cells were cultured on poly-L-lysine/fibronectin-coated chamber glass (4-well or 8-well, Watson) containing the respective MSP-1 differentiation media to induce differentiation into astrocytes or neurons. After the treatment, the cells were fixed with 4% PFA for 10 minutes and washed with TBS for 10 minutes. Subsequently, the cells were treated with 5% BSA diluted in 0.05% TBS-X at room temperature for 30 minutes, and then incubated overnight at 4°C in a blocking solution containing primary antibodies. The primary antibodies used in this study were anti-GFAP (mouse, 1:1000, Sigma), anti-AT8 (mouse, 1:1000, Thermo Fisher), anti-P-RIPK1 (mouse, 1:1000, Proteintech), anti-P-IKK (rabbit, 1:500, Cell Signaling), anti-Phospho-P65 (rabbit, 1:500, Cell Signaling), and anti-P-MLKL (rabbit, 1:2000, Abcam). The cells were then washed three times with TBS for 5 minutes each, and incubated with secondary antibodies at room temperature for 2 hours. The secondary antibodies used in this study were anti-mouse IgG donkey Alexa 488 (1: 1000, Molecular Probes) and anti-rabbit IgG donkey Alexa 568 (1:1000, Molecular Probes). After washing with TBS for 5 minutes, the sections were incubated with DAPI (1:10,000, Sigma) in TBS for 5 minutes, and then washed with TBS 2 times for 5 minutes each. The cells were visualized using a confocal microscope (FV3000-L4EN-TN21, Olympus). The images were analyzed and quantified using ImageJ.

### (Analysis of immunostaining images)

Microscopic images were analyzed using the ImageJ software. For AT8, P-RIPK1, and P-MLKL staining, staining intensity was calculated. Data are presented as relative intensity (ratio to control) ± S.E.M. for each group. For P-P65 staining, only the staining intensity in the nucleus was calculated, and the data are presented as relative intensity ± S.E.M. for each group. At least 100 cells for each group were analyzed from at least 10 random images.

### (RNA sequencing)

Total RNA was isolated from isolated astrocytes and astrocytes differentiated from MSP-1 cells using RNeasy Mini Kit (Qiagen). RNA samples in an amount of 1 ng (isolated astrocytes) or 10 ng (cultured astrocytes) were pretreated using the SMART-seq Stranded Kit (Takara Bio, Japan). Sequencing was performed using Illumina NovaSeq 6000 (Illumina, USA). Gene expression levels were evaluated using transcripts per million (TPM) data, and differentially expressed genes (DEGs) were identified using the DESeq2 package in the R environment. DEGs were further used for gene ontology (GO) analysis and Kyoto Encyclopedia of Genes and Genomes (KEGG) pathway analysis using the David analysis (https://david.nciferf.gov/). Gene set enrichment analysis (GSEA) was performed based on TPM data using the GESA software (UC San Diego and Broad Institute). The figures were generated using the ggplot2 package in the R environment.

### (Real-time quantitative PCR (RT-qPCR))

Reverse transcription reactions for cDNA synthesis were performed on the isolated RNA samples (500 ng) using SuperScript^{™} III Reverse Transcriptase (Thermo Fisher) according to the manufacturer's instructions. RT-qPCR was performed by using TP-850 (Takara Bio) and TB Green Taq (Takara Bio) with the following protocol: initial denaturation at 95°C for 30 seconds, followed by 40 cycles of 5 seconds at 95°C and 30 seconds at 60°C. All the samples were run in duplicate, and β-actin gene was used as the reference gene. Analysis was performed by the 2-ΔΔ-Ct method, and the results are expressed as fold changes. Gene sequences for RT-qPCR were designed by using Primer 3 (following table).

**[Table 1]**

| **Gene** | **Forward Primer** | **SEQ ID NO** | **Reverse Primer** | **SEQ ID NO** |
|---|---|---|---|---|
| C3 | GACGCCACTATGTCCATCCT | 1 | TACTCCAGAGGCCAGCAGTT | 2 |
| Ccl2 | CAGGTCCCTGTCATGCTTCT | 3 | CGTTAACTGCATCTGGCTGA | 4 |
| Cxcl1 | TGTTGTGCGAAAAGAAGTGC | 5 | TACAAACACAGCCTCCCACA | 6 |
| iNOS | AGACCTCAACAGAGCCCTCA | 7 | GGCTGGACTTTTCACTCTGC | 8 |

### [Results]

### In in vitro tauopathy cell culture model, astrocytes C3 induce necroptosis of neurons

First, to investigate the effects of activated astrocytes on neurodegeneration, a new cell culture model was established by using the MSP-1 cells, an immortalized neural stem cell line. The MSP-1 cells lack p53 and can proliferate indefinitely without undergoing apoptosis. The MSP-1 cells were induced to differentiate into neurons and astrocytes, and MSP-1 neurons were treated with the culture medium of MSP-1 astrocytes induced with TNF-α (Fig. 4). TNF-α significantly activated the NF-κB signaling pathway in the MSP-1 astrocytes, leading to nuclear accumulation of p-p65 (see Fig. 2B, Marge). RNA sequencing (RNA-Seq) revealed that the expressions of inflammatory factors such as C3, Ccl2, and Ccl7 increased following the TNF-α treatment (see Fig. 1D), and GESA analysis identified complement activation, which is thought to be associated with NF-κB activity (Fig. 1D) (see Fig. 2A, bottom left, first chart). Further, the increase in expressions of inflammatory factors, as represented by C3, following the TNF-α treatment was also confirmed by RT-PCR (see Fig. 1D). On the other hand, in the MSP-1 astrocytes pretreated with anserine, NF-κB activity and complement activation were reduced, and consequently, the expressions of inflammatory factors such as C3, which were observed to increase in the TNF-α-treated group, decreased (Fig. 5A). These results suggest that anserine suppresses astrocyte activation and C3 secretion.

Surprisingly, the MSP-1 neurons treated with the culture medium from the TNF-α group (TNF CM) then showed increased tau-phosphorylation, as revealed by AT-8 staining, while the MSP-1 neurons treated with the culture medium from the TNF-α + anserine group (anserine CM) did not exhibit such p-tau expression (Fig. 4, AT8/Dapi). Staining using necroptosis markers revealed that neurons treated with TNF-α CM exhibited high intensities of P-RIPK1 and P-MLKL (P-MLKL/Dapi and P-MLKL intensity in Fig. 4, and Fig. 5B), indicating activation of necroptosis. In contrast, direct treatment of MSP-1 neurons with TNF-α did not induce P-MLKL expression (Figs. 5C and 5D).

Necroptosis was induced by neurotoxic factors secreted from activated astrocytes. In the MSP-1 neurons, the intensities ofAT8, P-RIPK1, and P-MLKL were significantly lower. In particular, the MSP-1 neurons treated with anserine CM showed significant decrease in the intensities of AT8, P-RIPK1, and P-MLKL (p-MLKL intensity in Fig. 4). C3 has been reported to be involved in neuronal degeneration. Therefore, the role of C3 in necroptosis in MSP-1 neurons was investigated.

Since C3 is thought to affect target cells via C3-C3aR signaling, the action of C3 was inhibited using a C3aR antagonist. Interestingly, p-MLKL intensity decreased significantly to the same level as in the group treated with the necroptosis inhibitor, necrostatin-1 (Nec-1) (p-MLKL intensity in Fig. 4). These results suggest that C3 secretion from astrocytes may play a critical role in astrocyte-mediated neuronal necroptosis.

In the MSP-1 astrocytes treated with TNF-α, increases in RIPK1 and phosphorylated IKK complexes were observed; however, the addition of anserine reduced the expression of the phosphorylated IKK complex (Fig. 6). It is believed that anserine suppresses the neurotoxicity of TNF-α-induced astrocytes in vitro. Consequently, C3 release from astrocytes may be suppressed, and necroptosis in neurons may be suppressed.

### [Sequences listed in Sequence Listing]

SEQ ID NO: 1, PCR forward primer for C3
SEQ ID NO: 2, PCR reverse primer for C3
SEQ ID NO: 3, PCR forward primer for Ccl2
SEQ ID NO: 4, PCR reverse primer for Ccl2
SEQ ID NO: 5, PCR forward primer for Cxcl1
SEQ ID NO: 6, PCR reverse primer for Cxcl1
SEQ ID NO: 7, PCR forward primer for iNOS
SEQ ID NO: 8, PCR reverse primer for iNOS

## Claims

1. An agent for treating a disease or condition that is improved by regulating necroptosis, the agent comprising a compound represented by the following formula I or formula II, or a pharmaceutically acceptable salt thereof. 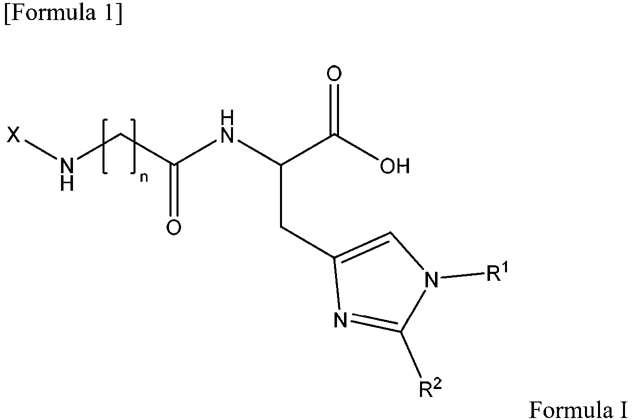 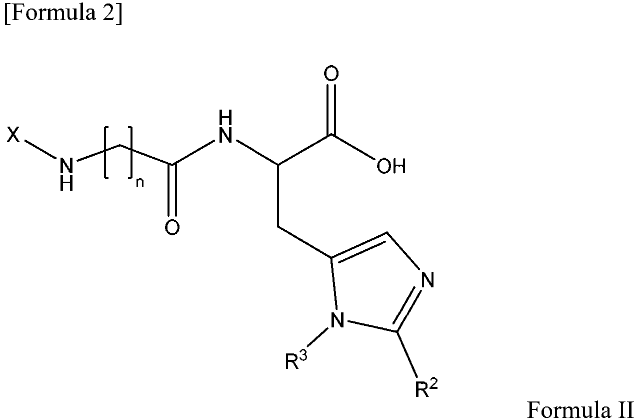 (in the formulas I and II, R¹, R²,and R³are independently H or C₁₋₆ alkyl, n is an integer of 1 to 6; and X is H or COR⁴, where R⁴ is H, C₁₋₆alkyl, benzyl (which may be substituted), or H₂C=CH-).

2. The agent according to claim 1, wherein necroptosis is neuronal necroptosis.

3. The agent according to claim 1, which regulates necroptosis-related responses in astrocytes.

4. The agent according to claim 1, which suppresses an increase in the expression level of any one selected from C3, Ccl2, Ccl7, Cxcl1, and iNOS.

5. The agent according to claim 1, which increases expression level of milk fat globule-EGF factor 8 protein (Mfge8).

6. The agent according to claim 1, which is for treating a subject carrying the type 4 allele of apolipoprotein E (APOE4) gene.

7. The agent according to any one of claims 1 to 6, wherein the compound represented by the formula I or formula II or a pharmaceutically acceptable salt thereof is a compound represented by the formula II or a pharmaceutically acceptable salt thereof, wherein R² is H, R³ is CH₃, and n is 2.

8. A method for screening for compounds that regulate necroptosis, using a compound represented by the formula I or formula II defined in claim 1 as a lead compound.

9. An inflammatory astrocyte obtainable by treating, with TNF-α, astrocytes obtained by inducing differentiation of mouse striatal precursor-1 (MSP-1) cells.

10. A method for screening for compounds that regulate necroptosis, using the inflammatory astrocyte according to claim 9.

11. The method according to claim 10, which comprises treating the inflammatory astrocyte according to claim 9 with candidate compounds, and selecting compounds from the candidate compounds based on whether increase in expression level of a necroptosis-inducing factor is regulated.

12. The method according to claim 10 or 11, which is for screening for compounds that regulate neuronal necroptosis.

13. An agent for regulating necroptosis, the agent comprising a compound represented by the formula I or formula II defined in claim 1, or a pharmaceutically acceptable salt thereof.

14. The agent according to any one of claims 1 to 6, which further has at least one of functions of inhibiting the NF-κB signaling pathway and inhibiting IKK activation.
